# EUROPÄISCHE PATENTANMELDUNG

(11) **EP 4 817 569 A1**
(43) Veröffentlichungstag der Anmeldung: **07.10.2026**
(21) Anmeldenummer: 26170282.3
(22) Anmeldetag: 01.04.2026
(51) Int. Cl.: A61F 7/00, A61G 7/057

(54) **TEMPERIERVORRICHTUNG ZUM TEMPERIEREN EINES LEBEWESENS**

(30) Priorität: 04.04.2025 AT 502402025
(71) Anmelder: Kohlbrat & Bunz Gesellschaft m.b.H, 5550 Radstadt (AT)
(72) Erfinder: Graf, Michael, 5584 Zederhaus (AT)
(74) Vertreter: Torggler & Hofmann Patentanwälte - Innsbruck

(57) **Zusammenfassung**

Temperiervorrichtung (1) zum Temperieren eines Lebewesens, insbesondere eines Menschen, umfassend eine Hülle (2) mit einer ersten Seite (2a) zur Auflage eines Lebewesens, und wenigstens ein Temperierelement (3), welches innerhalb der Hülle (2) angeordnet ist, wobei mittels des wenigstens einen Temperierelements (3) ein Wärmestrom erzeugbar ist, wobei die Temperiervorrichtung (1) wenigstens ein innerhalb der Hülle angeordnetes Wärmeübertragungselement (4) umfasst, mittels welchem der von dem wenigstens einen Temperierelement (3) erzeugte Wärmestrom auf die erste Seite (2a) der Hülle (2) übertragbar ist, um ein auf der Temperiervorrichtung (1) befindliches Lebewesen zu wärmen und/oder zu kühlen.

## Beschreibung

Die vorliegende Erfindung betrifft eine Temperiervorrichtung zum Temperieren eines Lebewesens, insbesondere eines Menschen, umfassend eine Hülle mit einer ersten Seite zur Auflage eines Lebewesens, und wenigstens ein Temperierelement, welches innerhalb der Hülle angeordnet ist, wobei mittels des wenigstens einen Temperierelements ein Wärmestrom erzeugbar ist. Zudem betrifft die Erfindung auch eine Vorrichtung mit einem solchen Wärmeelement und eine Anordnung aus einem solchen Wärmeelement und einer Auflagevorrichtung für ein Lebewesen.

Ein Wärmestrom ist dabei im physikalischen Sinne als die Menge an Wärme, welche von einem Ort hoher Temperatur zu einem Ort niedriger Temperatur übertragen wird, zu verstehen. Je nach Richtung des Wärmestroms kann der durch das Temperierelement erzeugte Wärmestrom demnach eine Erwärmung oder eine Abkühlung zur Folge haben.

Gattungsgemäße Temperiervorrichtungen sind bereits hinreichend aus dem Stand der Technik bekannt, beispielsweise aus der DE 10 2015 007 526. Diese Schrift offenbart eine temperaturregulierende Tragenauflage mit einer Hülle. Dabei ist unmittelbar unter einer PVC-Deckschicht, auf welche ein Lebewesen zur Auflage kommen kann, ein Temperierelement in Form einer Carbon-Heizmatte angeordnet, über welche das Lebewesen auf der Auflage gewärmt werden kann. Die Carbon-Heizmatte erstreckt sich dabei über einen Großteil der Fläche der Temperiervorrichtung.

Nachteilig bei solchen Temperiervorrichtungen ist, dass mittels des in der Hülle angeordenten Temperierelements keine Kühlung eines Lebewesens erreicht werden kann. Zudem sind großflächige Temperierelemente notwendig, um das auf der Auflage befindliche Lebewesen hinreichend temperieren zu können. Solche großflächigen Temperierelemente können aber kostenintensiv und auch anfällig für Beschädigungen sein, weshalb die genannte DE 10 2015 007 526 eine zusätzliche Stabilisierungsschicht für das Temperierelement vorsieht.

Zudem ist es auch nicht möglich, einen Innenraum einer Temperiervorrichtung nach DE 10 2015 007 526 zu evakuieren, da diese eine über einen Reißverschluss öffenbare Hülle aufweist. Das kann jedoch einige Vorteile mit sich bringen, welche im Folgenden noch näher beschrieben werden.

Die Aufgabe der gegenständlichen Erfindung besteht darin, eine gegenüber dem Stand der Technik verbesserte Temperiervorrichtung, welche die vorgenannten Nachteile zumindest teilweise vermeidet, sowie eine Vorrichtung mit einem solchen Wärmeelement und eine Anordnung aus einem solchen Wärmeelement und einer Auflagevorrichtung für ein Lebewesen anzugeben.

Diese Aufgaben werden gelöst durch die Merkmale der Ansprüche 1, 14 und 15.

Erfindungsgemäß ist bei einer wie oben beschriebenen Temperiervorrichtung vorgesehen, dass die Temperiervorrichtung wenigstens ein innerhalb der Hülle angeordnetes Wärmeübertragungselement umfasst, mittels welchem der von dem wenigstens einen Temperierelement erzeugte Wärmestrom auf die erste Seite der Hülle übertragbar ist, um ein auf der Temperiervorrichtung befindliches Lebewesen zu wärmen und/oder zu kühlen.

Dadurch ist es möglich, über das Temperierelement einen Wärmestrom bereitzustellen, welcher eine wärmende und/oder auch eine kühlende Wirkung auf die erste Seite der Hülle hat. Demnach kann ein auf der Hülle befindliches Lebewesen gewärmt und/oder gekühlt werden.

Ein erfindungsgemäßes Wärmeübertragungselement hat den Vorteil, dass kein großflächiges Temperierelement vorgesehen werden muss. Damit können kleine Temperierelemente vorgesehen werden, welche kostengünstig und wenig anfällig für Beschädigungen sind. Auch eine zusätzliche Stabilisierungsschicht kann eingespart werden.

Betreffend ein kühlend wirkendes Temperierelement ermöglicht ein erfindungsgemäßes Wärmeübertragungselement überhaupt erst eine sinnvolle Umsetzung einer Temperiervorrichtung mir ausreichender Kühlleistung. Flächige Temperierelemente, welche eine kühlende Wirkung erzeugen können und für den Einsatz in gattungsgemäßen Temperiervorrichtungen geeignet sind, sind nämlich nicht bekannt oder zu teuer.

Hinsichtlich einer erfindungsgemäßen Vorrichtung ist vorgesehen, dass die Temperiervorrichtung innerhalb der Vorrichtung angeordnet ist. Die Temperiervorrichtung ist also in die Vorrichtung integriert, was eine einfache Handhabung der Vorrichtung zur Folge hat.

Bei einer erfindungsgemäßen Anordnung ist vorgesehen, dass die Temperiervorrichtung auf einer dem Lebewesen zugewandten Seite der Auflagevorrichtung angeordnet ist. Die Temperiervorrichtung dient also als Auflage zwischen einem Lebewesen und der Auflagevorrichtung.

Weitere bevorzugte Ausführungsformen sind in den abhängigen Ansprüchen definiert.

Das Wärmeübertragungselement kann mit dem Temperierelement verbunden sein, um einen einfachen Wärmeübergang von dem Temperierelement auf das Wärmeübertragungselement zu erreichen. Bevorzugt kann dieser Wärmeübergang mittels Wärmeleitung und/oder Wärmestrahlung erfolgen.

Gemäß einem Ausführungsbeispiel der Erfindung kann vorgesehen sein, dass die Hülle wenigstens zwei flächige Materialbahnen aufweist, zwischen denen zumindest ein Innenraum ausgebildet ist, wobei die zumindest zwei Materialbahnen zumindest entlang ihrer jeweiligen Außenkanten, vorzugsweise unlösbar, miteinander verbunden, vorzugsweise verschweißt, sind.

Das stellt eine einfache und kostengünstige Möglichkeit zur Umsetzung einer erfindungsgemäßen Hülle dar. Insbesondere wenn die zwei Materialbahnen an ihren Außenkanten unlösbar verbunden sind, lässt sich eine geschlossene Hülle einfach bereitstellen.

In anderen Worten kann die Hülle frei von Öffnungen, welche eine Entnahme des wenigstens einen Heizelements und der wenigstens einen Energiequelle ermöglichen. Die Hülle kann also, mit Ausnahme kleinerer Öffnungen für beispielsweise ein Ventil zum Evakuieren eines Innenraums der Hülle oder eine Schnittstelle für eine externe Energiequelle, im Wesentlichen vollständig geschlossen sein.

Dies ermöglicht es, eine Hülle bereitzustellen, welche wasser- und gasdicht ausgebildet sowie evakuierbar ist, da keine Öffnungen oder dergleichen für eine eventuelle Entnahme des Temperierelements oder einer anderen innerhalb der Hülle angeordneten Komponente vorgesehen werden muss.

Die Materialbahnen können aus einem Kunststoff oder dergleichen bestehen. Es ist auch denkbar, dass die Materialbahnen aus einem Verbundmaterial bestehen, beispielsweise aus beschichtetem Stoff.

Jedenfalls sollten die Materialien für den Einsatz im medizinischen Bereich geeignet und/oder wasser- und gasdicht ausgeführt sein.

Vorteilhafterweise kann vorgesehen sein, dass das wenigstens eine Übertragungselement unmittelbar an einer ersten Materialbahn der zumindest zwei Materialbahnen angeordnet ist, wobei die erste Materialbahn die erste Seite der wenigstens einen Hülle ausbildet.

Somit befindet sich nur eine der Materialbahnen zwischen dem Heizelement und einem Lebewesen, sodass eine gute Wärmeübertragung von dem Temperierelement auf das Lebewesen oder vice versa sichergestellt werden kann.

Es kann weiters vorgesehen sein, dass die Hülle wasserdicht und/oder gasdicht und/oder elastisch ausgebildet ist.

Eine wasser- und gasdichte Ausbildung der Hülle erlaubt eine einfache und tiefgehende Reinigung der Hülle. Zudem sind Komponenten im Inneren der Hülle vor schädlichen Umgebungseinflüssen bestmöglich geschützt.

Eine elastische Ausbildung der Hülle erlaubt eine gute Anpassung der Hülle an die Form eines Lebewesens. Dahingehend kann vorteilhafterweise auch vorgesehen sein, dass das Wärmeübertragungselement elastisch ausgebildet ist.

In manchen Ausführungsforme kann auch das Temperierelement elastisch ausgeführt sein, auch wenn dies aufgrund des Wärmeübertragungselements nicht notwendig ist.

Bevorzugt kann vorgesehen sein, dass das Temperierelement wenigstens ein Peltier-Element umfasst, vorzugsweise durch wenigstens ein Peltier-Element ausgebildet ist.

Ein Peltier-Element erzeugt bei Stromdurchfluss eine Temperaturdifferenz und so einen Wärmestrom. In anderen Worten erfährt bei Stromdurchfluss eine Seite des Peltier-Elements eine Abkühlung, während eine andere Seite des Peltier-Elements erwärmt wird.

Demgemäß kann das Peltier-Element sowohl zur Kühlung als auch zur Erwärmung eines auf der Temperiervorrichtung befindlichen Lebewesens erfolgen, abhängig davon, von welcher Seite des Peltier-Elements der Wärmestrom von dem Wärmeleitelement abgegriffen wird. Das Peltier-Element kann also eine Doppelfunktion erfüllen.

Zudem weisen Peltier-Elemente eine geringe Größe, ein geringes Gewicht und, in der Größe, wie sie für eine gattungsgemäße Temperiervorrichtung erforderlich sind, geringe Kosten auf.

Dadurch lassen sich Peltier-Elemente einfach innerhalb einer Hülle einer Temperiervorrichtung anordnen, was bei herkömmlichen Kältemaschinen nicht möglich wäre. Das ist besonders vorteilhaft, da somit keine externen Geräte (eventuell mit Ausnahme eine Energieversorgung) zum Betrieb der Temperiervorrichtung notwendig sind.

Bezüglich eines Peltier-Elements kann auch noch vorgesehen sein, dass das wenigstens eine Wärmeübertragungselement mit einer ersten Seite und/oder einer zweiten Seite des Peltier-Elements verbunden und/oder verbindbar ist.

Es ist auch denkbar, dass das Wärmeübertragungselement, je nach dem, ob ein Lebewesen gekühlt oder gewärmt werden soll, mit der ersten oder der zweiten Seite des Peltier-Elements verbunden ist.

Dadurch kann die Doppelfunktion des Peltier-Elements ausgenutzt werden.

Gemäß einer weiteren Ausführungsform der Erfindung können wenigstens zwei Temperierelemente vorgesehen sein, wobei ein erstes der zwei Temperierelemente für das Wärmen eines Lebewesens und das andere der zwei Temperierelemente für das Kühlen eines Lebewesens zuständig ist.

Dadurch kann sowohl für das Kühlen als auch für das Wärmem das jeweils am besten geeignete Temperierelement gewählt werden.

Sind beide Temperierelemente als Peltier-Elemente ausgebildet, so kann vorgesehen sein, dass ein erstes der beiden Peltier-Elemente mit einer ersten Seite und das zweite der beiden Peltier-Elemente mit einer zweiten Seite mit dem Wärmeübertragungselement in Kontakt steht bzw. verbunden ist. Dadurch kann mittels eines ersten Peltier-Elements gekühlt und mittels des zweiten Peltier-Elements gewärmt werden.

Es ist auch denkbar, dass bei zwei Temperierelementen zwei Wärmeübertragungselemente vorgesehen sind.

Bevorzugt kann vorgesehen sein, dass das Wärmeübertragungselement
- eine luftundurchlässige obere Lage,
- eine luftundurchlässige untere Lage, die der oberen Lage gegenüber liegt,
- mindestens eine zwischen oberer Lage und unterer Lage angeordnete und luftundurchlässige Zwischenlage, und
- ein Transportmittel zum Transport eines Gases, vorzugsweise Luft, durch die Zwischenlage
umfasst, wobei der von dem wenigstens einen Temperierelement erzeugte Wärmestrom über das durch die Zwischenlage transportierbare Gas auf die erste Seite der Hülle übertragbar ist.

Das stellt eine vorteilhafte Möglichkeit zur Umsetzung eines Wärmeübertragungselements dar. Dabei gibt das Temperierelement Wärme an ein Gas ab bzw. entzieht dem Gas Wärme ("gibt Kälte ab"). Das vom Temperierelement erwärmte bzw. gekühlte Gas wird dann über das Transportmittel durch die Zwischenlage zur ersten Seite der Hülle befördert, wo es Wärme an die erste Seite abgibt oder dieser Wärme entzieht, vorzugsweise über Wärmeleitung zwischen dem Übertragungsgelement und der ersten Seite.

Das Transportmittel kann beispielsweise einen Lüfter umfassen.

Das Gas ist bevorzugt Luft. Es ist aber auch denkbar, dass andere Gase mit einer besseren Wärmeleitfähigkeit zum Einsatz kommen.

Es kann auch vorgesehen sein, dass die Hülle eine Eintrittsöffnung zum Eintritt von Luft in die Hülle und eine Austrittsöffnung zum Austritt von Luft aus der Hülle aufweist.

Dabei kann vorgesehen sein, dass Luft über das Transportmittel durch die Eintrittsöffnung in die Hülle, durch das Wärmeübertragungselement und anschließen durch die Austrittsöffnung aus der Hülle gefördert wird.

Dadurch ist es möglich "verbrauchte" Luft, welche ihre Wärme an die erste Seite abgegeben hat oder Wärme von der ersten Seite aufgenommen hat, aus der Temperiervorrichtung hinauszubefördern. Gleichzeitig kann "frische" Umgebungsluft zugeführt werden, welche dann über das Temperierelement erwärmt und/oder abgekühlt werden kann.

Es ist jedoch auch denkbar, dass sich das Gas in einem geschlossenen Kreislauf befindet, wobei das Gas, welches seine Wärme an die erste Seite abgegeben oder Wärme von der ersten Seite aufgenommen hat, wieder dem Temperierelement zum Erwärmen oder Abkühlen zugeführt wird. Das ist insbesondere interessant, wenn es sich bei dem Gas nicht um Luft handelt.

Bevorzugt kann vorgesehen sein, dass die obere Lage, die untere Lage und die Zwischenlage ein Abstandsgewirke bilden.

Damit kann das Wärmeübertragungselement auch als Polsterung für das Lebewesen wirken, und so den Liegekomfort des Lebewesens erhöhen. Das Wärmeübertragungselement hat also eine Doppelfunktion inne.

Gemäß einem weiteren Ausführungsbeispiel kann vorgesehen sein, dass das wenigstens eine Wärmeübertragungselement mit der ersten Seite und dem wenigstens einen Temperierelement verbunden ist, sodass der von dem wenigstens einen Temperierelement erzeugte Wärmestrom mittels Wärmeleitung auf das wenigstens eine Wärmeübertragungselement und vom wenigstens einen Wärmeübertragungselement auf die erste Seite der Hülle übertragbar ist.

Das stellt eine einfache Umsetzung eines Wärmeübertragungselements dar. Insbesondere kann das Wärmeübertragungselement in dieser Ausführungsform als Folie ausgebildet sein.

Vorteilhafterweise kann dabei vorgesehen sein, dass das Wärmeübertragungselement einen Wärmeleitkoeffizient von mindestens 1 W/mK aufweist. Selbstverständlich können auch höhere Wärmeleitkoeffizienten vorgesehen sein, da sich mit steigenden Wärmeleitkoeffizienten auch die Effizienz der Wärmeleitung erhöht.

Es kann vorgesehen sein, dass das wenigstens eine Wärmeübertragungselement ein wärmeleitfähiges Gel und/oder eine wärmeleitfähige Flüssigkeit und/oder ein Metall umfasst.

Umfasst das Wärmeübertragungselement ein wärmeleitfähiges Gel und/oder eine wärmeleitfähige Flüssigkeit, so kann es eine Einhausung umfassen, in welcher das wärmeleitfähige Gel und/oder die wärmeleitfähige Flüssigkeit angeordnet ist.

Die Einhausung selbst kann dabei auch wärmeleitfähig sein.

Das Temperierelement kann direkt mit dem wärmeleitfähigen Gel und/oder der wärmeleitfähigen Flüssigkeit oder mit der Einhausung in Kontakt stehen.

Bevorzugt kann vorgesehen sein, dass sich das wenigstens eine Wärmeübertragungselement über zumindest 30%, vorzugsweise über zumindest 50%, der Fläche der Hülle erstreckt und/oder wobei sich der wenigstens eine Temperierelement über maximal 25 % der Fläche der Hülle erstreckt.

Das Wärmeübertragungselement kann sich insbesondere auch über zumindest 60 %, 70 %, 80 %, 85 %, 90 %, 95 % oder 100 % der Fläche erstrecken.

Das Wärmeübertragungselement kann auch eine Fläche aufweisen, wobei die Fläche des Wärmeübertragungselements zumindest 30 %, 50 % 60 %, 70 %, 80 %, 85 %, 90 %, 95 % oder 100 % der Fläche der Hülle beträgt.

Damit kann sichergestellt werden, dass ein auf der Temperiervorrichtung befindliches Lebewesen ausreichend und an allen Stellen temperiert werden kann.

Das Temperierelement kann sich insbesondere auch über maximal 20%, 15 %, 10 % oder 5% der Fläche der Hülle erstrecken.

Das Temperierelement muss also nicht großflächig ausgebildet sondern kann auch verhältnismäßig klein sein. Dadurch kann ein kleines und weniger für Beschädigungen anfälliges Temperierelement bereitgestellt werden.

Weiters kann auch vorgesehen sein, dass das Wärmeelement eine Isolierung, vorzugsweise in Form eines Granulats und/oder eines Abstandgewirkes oder -gewebes, aufweist.

Die Isolierung dient einerseits dazu, eine ungewünschte Wärmeabstrahlung in eine Richtung, in welche kein Lebewesen mit dem Wärmeübertragungselement bzw. der ersten Seite der Hülle in Kontakt ist, zu verhindern. Andererseits kann die Isolierung auch als Stabilisierung des Wärmeübertragungselement dienen, auch wenn dies nicht zwingend notwendig ist.

Besonders bevorzugt kann vorgesehen sein, dass wenigstens ein Innenraum der Hülle, vorzugsweise über ein an der Hülle angeordnetes Ventil, evakuierbar ist. In anderen Worten ist im Innenraum der Hülle ein Vakuum erzeugbar.

Daraus ergeben sich einige Vorteile gegenüber herkömmlichen Temperiervorrichtungen. Einerseits ist das Wärmeübertragungselement durch die Evakuierung des Innenraums im Wesentlichen vollflächig an die Hülle angeformt, sodass hier ein guter Wärmeübergang zwischen Wärmeübertragungselement und Hülle, und in weiterer Folge zwischen Hülle und Lebewesen, gegeben ist.

Andererseits kann das Wärmeübertragungselement dadurch gut an die Kontur eines Lebewesens (beispielsweise Kopf oder Schulter) angeformt werden. Dadurch wir eine größtmögliche direkte Auflage des Wärmeübertragungselement an dem Lebewesen erreicht, wodurch ein effektives Wärmen des Lebewesens erreichbar ist. Dieser Effekt wird zusätzlich verstärkt, wenn eine Isolierung vorgesehen ist, welche ein Abstrahlen von Wärme von einem Lebewesen weg verhindert bzw. verringert werden kann. Das Anformen des Wärmeübertragungselement an dem Lebewesen führt zudem zu einer gleichmäßigen Druckverteilung im Bereich der Temperiervorrichtung, sodass Druckstellen vorgebeugt werden kann.

Das Anformen kann dabei analog zu Vakuummatratzen erfolgen. D.h. das Lebewesen wird auf die nicht evakuierte Temperiervorrichtung gelegt, wodurch sich dieses über die Isolierung in Form von Granulat oder eines Abstandgewirkes oder -gewebes an die Kontur des Lebewesens anpasst. Beim Evakuieren der Temperiervorrichtung wird die Temperiervorrichtung dann durch die Isolierung in dieser angepassten Form festgelegt.

Vorteilhafterweise ist zum Evakuieren ein entsprechendes Ventil vorgesehen, über welches der Innenraum der Hülle auch wieder belüftbar ist. Das Vakuum kann über bereits hinreichend bekannte Vorrichtung erzeugt werden.

Vorteilhafterweise kann vorgesehen sein, dass die Temperiervorrichtung wenigstens eine Schnittstelle zur Versorgung des wenigstens einen Temperierelements mit Energie, vorzugsweise elektrischer Energie, aus einer externen Energiequelle umfasst.

Die Schnittstelle kann in einer Öffnung in der Hülle angeordnet oder aus der Hülle hinausgeführt sein. Es ist auch denkbar, dass die Schnittstelle innerhalb der Hülle angeordnet ist.

Die Hülle und die Schnittstelle können dabei derart ausgebildet sein, dass der Innenraum der Hülle weiterhin evakuierbar ist.

Die Schnittstelle kann beispielsweise ein Stecker oder eine Buchse sein. Die Schnittstelle kann aber auch in Form einer induktiven Schnittstelle zur induktiven Übertragung von Energie ausgebildet sein.

Eine externe Energiequelle ist eine gesondert von der Temperiervorrichtung ausgebildete und nicht zu dieser gehörenden Energiequelle. Dabei kann es sich beispielsweise um eine herkömmliche Steckdose, einen Stromanschluss in einem Fahrzeug oder auch einem mobilen Akkumulator handeln.

Gemäß einem weiteren Ausführungsbeispiel kann vorgesehen sein, dass die Temperiervorrichtung wenigstens einen Temperatursensor aufweist, wobei das Temperierelement in Abhängigkeit von Messwerten des wenigstens einen Temperatursensors regelbar ist.

Somit kann sichergestellt werden, dass das Lebewesen stets ideal über die Temperiervorrichtung temperiert wird.

Besonders bevorzugt ist dazu wenigstens eine Regelungsvorrichtung zur Regelung einer dem wenigstens einen Temperierelement zugeführten, vorzugsweise elektrischen, Energie in Abhängigkeit von Messwerten des wenigstens einen Temperatursensors vorgesehen.

Vorteilhafterweise kann die Regelungsvorrichtung dazu ausgebildet sein, die dem wenigstens einen Temperierelement zugeführte, vorzugsweise elektrische, Energie derart zu regeln, dass am Wärmeübertragungselement eine vordefinierte Temperatur nicht über- und/oder unterschritten wird.

Wichtig bei der Anwendung einer Temperiervorrichtung ist es, dass dem Lebewesen durch eine Wärmezufuhr keine Schäden wie Verbrennungen zugefügt werden. Es ist daher wichtig, die maximale Temperatur an der Temperiervorrichtung zu regeln. Als maximale Hautkontakt-Temperatur zur Vermeidung von Verbrennungen wird in der einschlägigen Literatur für Menschen eine Temperatur von 60°C für die Dauer von 5 Sekunden angeführt. Allerdings kann bei direkter Einwirkung auf die Haut beziehungsweise ein Gewebe bereits ab einer Temperatur von 45°C eine Schädigung von Zellen auftreten. Es kann daher vorteilhaft sein, die Temperatur auf unterhalb von 45°C zu begrenzen. Stützend auf klinischen Studien ist eine maximale Temperatur von 42°C erstrebenswert.

Es kann auch vorgesehen sein, dass der Temperatursensor an der ersten Seite der Hülle angeordnet ist und/oder wobei die Isolation an einer der ersten Seite gegenüberliegenden zweiten Seite der Hülle angeordnet ist. Diese zweite Seite ist dabei dem Lebewesen abgewandt.

Wie bereits ausgeführt, kann dadurch erreicht werden, dass ein größtmöglicher Teil der vom Temperierelement erzeugten Wärme bzw. Kälte dem Lebewesen möglichst direkt zugeführt werden kann. Somit können Verluste minimiert und die Effizienz der Temperiervorrichtung erhöht werden.

Es kann auch vorgesehen sein, dass die Temperiervorrichtung ein Betätigungselement zum Bedienen der Temperiervorrichtung aufweist. Über das Betätigungselement kann die Temperiervorrichtung beispielsweise ein- oder ausgeschaltet oder ein Soll-Temperatur-Wert, auf welchen ein Lebewesen erwärmt bzw. gekühlt werden soll, festgelegt werden.

Es kann auch vorgesehen sein, dass die Temperiervorrichtung wenigstens ein Befestigungselement zur Befestigung der Temperiervorrichtung an einer von der Temperiervorrichtung gesonderten Auflagevorrichtung für ein Lebewesen, insbesondere eine Krankentrage, aufweist.

Dadurch lässt sich die Temperiervorrichtung einfach an einer Auflagevorrichtung festlegen. Bei dem wenigstens einen Befestigungselement kann es sich beispielsweise um Ösen, einer Begurtung oder andere geeigneten Mitteln handeln.

Eine erfindungsgemäße Temperiervorrichtung kann auf verschiedensten Arten zum Einsatz kommen, beispielsweise als Kopfkissen, Wärmekissen oder Sitzauflage. Die Temperiervorrichtung kann aber auch in solche Produkte integriert sein. Zudem sind Vakuummatratzen und Halskrausen mit integrierter Temperiervorrichtung denkbar.

Weitere Einzelheiten und Vorteile bevorzugter Ausführungsformen der Erfindung werden anhand der Figurenbeschreibung unter Bezugnahme auf die Zeichnungen im Folgenden näher erläutert. Darin zeigen:
- Fig. 1: eine schematische Schnittdarstellung eines Ausführungsbeispiels einer Temperiervorrichtung,
- Fig. 2: eine schematische Schnittdarstellung eines weiteren Ausführungsbeispiels der Temperiervorrichtung,
- Fig. 3: eine schematische Schnittdarstellung eines weiteren Ausführungsbeispiels der Temperiervorrichtung,
- Fig. 4: eine schematische Schnittdarstellung eines weiteren Ausführungsbeispiels der Temperiervorrichtung,
- Fig. 5: eine schematische Ansicht eines Ausführungsbeispiels einer Vorrichtung, und
- Fig. 6: eine schematische Ansicht eines Ausführungsbeispiels einer Anordnung.

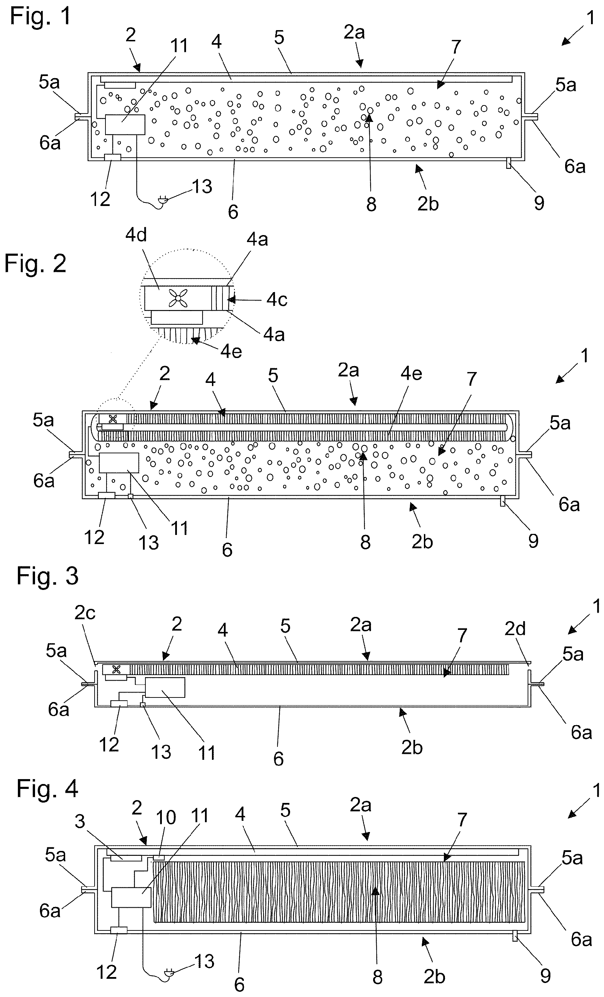

Die Fig. 1 zeigt eine schematische Schnittdarstellung eines Ausführungsbeispiels einer Temperiervorrichtung 1. Dabei ist eine Hülle 2 erkennbar, wobei die Hülle 2 eine erste Materialbahn 5 und eine zweite Material 6, welche entlang ihren Außenkanten 5a, 6a unlösbar miteinander verbunden sind, aufweist. Diese unlösbare Verbindung kann im gegenständlichen Ausführungsbeispiel durch Verschweißen der beiden Materialbahnen 5, 6 miteinander hergestellt werden. Die Hülle 2 weist eine erste Seite 2a und eine der ersten Seite gegenüberliegende zweite Seite 2b auf. Die erste Seite 2a ist dabei eine einem Lebewesen zuzuwendende Seite und die zweite Seite 2b eine einem Lebewesen abzuwendenden Seite.

Die Hülle 2 bildet einen Innenraum 7 aus, innerhalb welchem die Komponenten der Temperiervorrichtung 1 angeordnet sind. Die Temperiervorrichtung 1 kann so als in sich geschlossenes System ausgebildet sein, welches ob der wasser- und gasdichten Ausbildung der Hülle 2 vor Umwelteinflüssen geschützt ist.

Über das Ventil 9 ist eine Verbindung zum Innenraum 7 herstellbar, um diesen zu evakuieren bzw. nach erfolgter Evakuierung wieder zu belüften.

Zudem ist in diesem Ausführungseispiel eine Schnittstelle 13 in Form eines Kabels mit einem Stecker 13 aus der Hülle 2 herausgeführt. Ein Betätigungselement 12 ist in die zweite Materialbahn 6 der Hülle 2 integriert. Das hat jedoch keinen Einfluss auf die vorgenannten Eigenschaften der Hülle 2.

Im Innenraum 7 sind weiter ein Temperierelement 3, ein Wärmeübertragungselement 4 und eine Regelungsvorrichtung 11 zur Regelung bzw. Steuerung des Temperierelements 3 erkennbar.

Das Wärmeübertragungselement 4 ist unmittelbar an der ersten Materialbahn 5, und somit der ersten Seite 2a, angeordnet, um einen bestmöglichen Wärmeübergang vom Wärmeübertragungselement 4 zu einem Lebewesen zu ermöglichen.

Das Wärmeübertragungselement 4 ist in diesem Ausführungsbeispiel beispielsweise als Folie ausgebildet oder umfasst ein von einer Einhausung umschlossenes leitfähiges Gel oder Flüssigkeit.

In diesem Ausführungsbeispiel ist das Temperierelement 3 mit dem Wärmeübertragungselement 4 verbunden und überträgt so Wärme oder Kälte mittels Wärmeleitung an das Wärmeübertragungselement 4, welches wiederum mit der ersten Materialbahn 2a in Kontakt steht.

Es ist erkennbar, dass das Temperierelement 3 klein ausgeführt werden kann, das Wärmeübertragungselement 4 sich jedoch über einen Großteil der Fläche der Hülle 2 bzw. der ersten Materialbahn 5 erstreckt.

Weiters ist eine Isolierung 8 in Form eines Granulats erkennbar, welches den Innenraum 7 zumindest teilweise füllt. das Granulat kann dabei beispielsweis aus Polysterol bestehen. Die Isolierung 8 verringert eine Wärmeübertragung vom Wärmeübertragungselement 3 in Richtung der zweiten Seite 2b, wodurch an der ersten Seite 2a mehr Wärme bzw. Kälte zur Verfügung steht.

Über das Ventil 9 kann der Innenraum 7 evakuiert werden. Über die Isolierung 8 kann das Wärmeelement 1 dann nach Art einer Vakuummatratzen an die Konturen eines Lebewesens angeformt werden.

Die Schnittstelle 13 ist mit der Regelungsvorrichtung 10 verbunden. Die Regelungsvorrichtung 10 ist mit dem Betätigungselement 12 und dem Temperierelement 3 verbunden. Über die Regelungsvorrichtung 10 kann das Temperierelement 3 mit Energie versorgt und gesteuert und/oder geregelt werden, beispielsweise über Eingaben eines Benutzers über das Betätigungselement 12.

Die Fig. 2 zeigt eine schematische Schnittdarstellung eines weiteren Ausführungsbeispiels einer Temperiervorrichtung 1. Diese unterscheidet sich von dem Ausführungsbeispiel nach Fig. 1 im Wesentlichen durch die Ausbildung des Wärmeübertragungselements 4 und der Schnittstelle 13.

Das Wärmeübertragungselement 4 umfasst eine luftundurchlässige obere Lage 4a, eine luftundurchlässige untere Lage 4b und eine luftdurchlässige Zwischenlage 4c. Zudem ist ein Transportmittel 4d zum Transport eines Gases, vorzugsweise Luft, durch die Zwischenlage 4c erkennbar.

Das Temperierelement 3 ist mit dem Transportmittel 4 verbunden und gibt an dieses Wärme ab bzw. entzieht diesem Wärme. Über einen Lüfter des Transportmittels 4d kann ein Gas durch das Wärmeübertragungselement 4 befördert werden und so die mit dem Wärmeübertragungselement 4 in Kontakt stehende erste Materialbahn 5 erwärmen oder abkühlen.

Im vorliegenden Ausführungsbeispiele ist ein geschlossenes System vorgesehen. D.h. ein Gas kann, nachdem es über das Wärmeübertragungselement Wärme an die erste Materialbahn 5 abgegeben oder dieser Wärme entzogen hat, über eine Rückleitung 4e wieder dem Temperierelement 3 zugeführt und darin erwärmt oder abgekühlt werden.

Die obere Lage 4a, die untere Lage 4b und die Zwischenlage bilden in diesem Ausführungsbeispiel ein Abstandsgewirke aus.

Die Rückleitung 4e ist analog zum Wärmeübertragungselement 4 ausgebildet. Es kann aber auch eine davon verschiedene Ausbildung der Rückleitung 4e vorgesehen sein.

Die Schnittstelle 13 ist in diesem Ausführungsbeispiel nicht als Stecker, sondern als in die Hülle integrierte Buchse ausgebildet.

Die Fig. 3 zeigt eine schematische Schnittdarstellung eines weiteren Ausführungsbeispiels einer Temperiervorrichtung 1. Diese unterscheidet sich von dem Ausführungsbeispiel nach Fig. 2 im Wesentlichen durch, dass ein offenes System bei dem Wärmeübertragungselement 4 vorgesehen ist.

Die Hülle 2 weist demnach eine Eintrittsöffnung 2c zum Eintritt von Luft in den Innenraum 7 und eine Austrittsöffnung 2d zum Austritt von Luft aus dem Innenraum 7 auf.

Über das Transportmittel 4d kann also Luft in die Hülle 2 und in das Wärmeübertragungselement 4 transportiert und anschließend über die Austrittsöffnung 4d wieder aus der Hülle 2 gefördert werden. Es ist demnach keine Rückleitung 4e vorgesehen.

Eine Temperiervorrichtung 1 nach diesem Ausführungsbeispiel ist demnach nicht evakuierbar. Es ist deshalb auch keine Isolierung 8 in Form eines Granulats vorgesehen. Es ist allerdings denkbar, dass eine Isolierung 8 in Form eines Abstandgewirkes oder - gewebes ausgebildet ist, ähnlich dem folgenden Ausführungsbeispiel nach Fig. 4.

Die Fig. 4 zeigt eine schematische Schnittdarstellung eines weiteren Ausführungsbeispiels einer Temperiervorrichtung 1. Diese unterscheidet sich von dem Ausführungsbeispiel nach Fig. 1 im Wesentlichen durch, dass die Isolierung 8 in Form eines Abstandgewirkes oder -gewebes ausgebildet ist. Zudem ist auch ein Temperatursensor 10 ersichtlich, welcher die Temperatur am Wärmeübertragungselement 4 messen kann. Auf Basis dieser Messwerte kann die Regelungsvorrichtung 10 dann die dem Heizelement 3 zugeführte Energie regeln, um eine bestimmte Temperatur zu halten bzw. nicht zu überschreiten.

Ein solcher Temperatursensor 4 kann aber in jedem der gezeigten Ausführungsbeispiele vorgesehen sein.

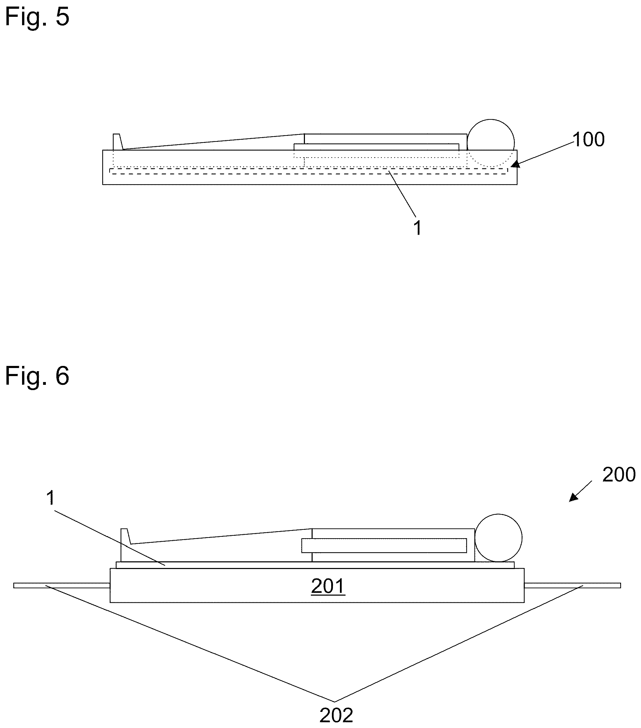

Die Fig. 5 zeigt eine schematische Ansicht eines Ausführungsbeispiels einer Vorrichtung 100. Die Vorrichtung 100 ist als Vakuummatratze ausgebildet. Es ist erkennbar, dass ein Mensch in der Vakuummatratze aufgenommen ist. Zudem ist ein Wärmeelement 1 angedeutet, welches innerhalb der Vorrichtung 100 angeordnet ist und über welches dem Menschen in der Vorrichtung 100 Wärme zugeführt werden kann.

Die Fig. 6 zeigt eine schematische Ansicht eines Ausführungsbeispiels einer Anordnung 200. Eine Auflagevorrichtung 201 kann beispielsweise als Krankentrage ausgebildet sein und Tragegriffe 202 aufweisen. Auf der Auflagevorrichtung 201 ist ein Wärmeelement 1 angeordnet, auf welchem wiederum ein Mensch liegen kann. Über das Wärmelement 1 kann dem Menschen auf der Auflagevorrichtung 200 Wärme zugeführt werden kann.

### Bezugszeichenliste:

- 1: Temperierelement
- 2: Hülle
2a erste Seite
2b zweite Seite
2c Eintrittsöffnung
2d Austrittsöffnung
- 3: Temperierelement
- 4: Wärmeübertragungselement
4a obere Lage
4b untere Lage
4c Zwischenlage
4d Transportmittel
4e Rückleitung
- 5: erste Materialbahn
5a Außenkante
- 6: zweite Materialbahn
6a Außenkante
6b Sichtfenster
- 7: Innenraum
- 8: Isolierung
- 9: Ventil
- 10: Temperatursensor
- 11: Regelungsvorrichtung
- 12: Betätigungselement
- 13: Schnittstelle
- 100: Vorrichtung
- 200: Anordnung
- 201: Auflagevorrichtung
- 202: Tragegriffe

## Patentansprüche

1. Temperiervorrichtung (1) zum Temperieren eines Lebewesens, insbesondere eines Menschen, umfassend
- eine Hülle (2) mit einer ersten Seite (2a) zur Auflage eines Lebewesens, und
- wenigstens ein Temperierelement (3), welches innerhalb der Hülle (2) angeordnet ist, wobei mittels des wenigstens einen Temperierelements (3) ein Wärmestrom erzeugbar ist,
**dadurch gekennzeichnet, dass** die Temperiervorrichtung (1) wenigstens ein innerhalb der Hülle angeordnetes Wärmeübertragungselement (4) umfasst, mittels welchem der von dem wenigstens einen Temperierelement (3) erzeugte Wärmestrom auf die erste Seite (2a) der Hülle (2) übertragbar ist, um ein auf der Temperiervorrichtung (1) befindliches Lebewesen zu wärmen und/oder zu kühlen.

2. Temperiervorrichtung (1) nach Anspruch 1, wobei die Hülle wenigstens zwei flächige Materialbahnen (5, 6) aufweist, zwischen denen zumindest ein Innenraum (7) ausgebildet ist, wobei die zumindest zwei Materialbahnen (5, 6) zumindest entlang ihrer jeweiligen Außenkanten (5a, 6a), vorzugsweise unlösbar, miteinander verbunden, vorzugsweise verschweißt, sind.

3. Temperiervorrichtung (1) nach Anspruch 2, wobei das wenigstens eine Wärmeübertragungselement (4) unmittelbar an einer ersten Materialbahn (5) der zumindest zwei Materialbahnen (5, 6) angeordnet ist, wobei die erste Materialbahn (5) die erste Seite (2a) der wenigstens einen Hülle (2) ausbildet.

4. Temperiervorrichtung (1) nach einem der Ansprüche 1 bis 3, wobei die Hülle (2) wasserdicht und/oder gasdicht und/oder elastisch ausgebildet ist.

5. Temperiervorrichtung (1) nach einem der Ansprüche 1 bis 4, wobei das Temperierelement (3) wenigstens ein Peltier-Element umfasst, vorzugsweise durch wenigstens ein Peltier-Element ausgebildet ist.

6. Temperiervorrichtung (1) nach einem der Ansprüche 1 bis 5, wobei das Wärmeübertragungselement (4)
- eine luftundurchlässige obere Lage (4a),
- eine luftundurchlässige untere Lage (4b), die der oberen Lage (4a) gegenüber liegt,
- mindestens eine zwischen oberer Lage (4a) und unterer Lage (4b) angeordnete und luftundurchlässige Zwischenlage (4c), und
- ein Transportmittel (4d) zum Transport eines Gases, vorzugsweise Luft, durch die Zwischenlage (4c)
umfasst, wobei der von dem wenigstens einen Temperierelement (3) erzeugte Wärmestrom über das durch die Zwischenlage (3c) transportierbare Gas auf die erste Seite (2a) der Hülle (2) übertragbar ist.

7. Temperiervorrichtung (1) nach Anspruch 6, wobei die obere Lage (4a), die untere Lage (4b) und die Zwischenlage (4c) ein Abstandsgewirke bilden.

8. Temperiervorrichtung (1) nach einem der Ansprüche 1 bis 5, wobei das wenigstens eine Wärmeübertragungselement (4) mit der ersten Seite (2a) und dem wenigstens einen Temperierelement (3) verbunden ist, sodass der von dem wenigstens einen Temperierelement (3) erzeugte Wärmestrom mittels Wärmeleitung auf das wenigstens eine Wärmeübertragungselement (4) und vom wenigstens einen Wärmeübertragungselement (4) auf die erste Seite (2a) der Hülle (2) übertragbar ist.

9. Temperiervorrichtung (1) nach Anspruch 8, wobei das Wärmeübertragungselement (4) einen Wärmeleitkoeffizient von mindestens 1 W/mK aufweist.

10. Temperiervorrichtung (1) nach Anspruch 9, wobei das wenigstens eine Wärmeübertragungselement (4) ein wärmeleitfähiges Gel und/oder eine wärmeleitfähige Flüssigkeit und/oder ein Metall umfasst.

11. Temperiervorrichtung (1) nach einem der Ansprüche 1 bis 7, wobei sich das wenigstens eine Wärmeübertragungselement (4) über zumindest 30 %, vorzugsweise über zumindest 50 %, der Fläche der Hülle (2) erstreckt und/oder wobei sich der wenigstens eine Temperierelement (3) über maximal 25 % der Fläche der Hülle (2) erstreckt.

12. Temperiervorrichtung (1) nach einem der Ansprüche 1 bis 11, wobei wenigstens ein Innenraum (7) der Hülle (2), vorzugsweise über ein an der Hülle (2) angeordnetes Ventil (9), evakuierbar ist.

13. Temperiervorrichtung (1) nach einem der Ansprüche 1 bis 12, wobei die Temperiervorrichtung (1) wenigstens eine Schnittstelle (13) zur Versorgung des wenigstens einen Temperierelements (1) mit Energie, vorzugsweise elektrischer Energie, aus einer externen Energiequelle umfasst.

14. Vorrichtung (100), insbesondere Vakuummatratze, zur Stützung und/oder Stabilisierung wenigstens eines Lebewesens, insbesondere eines Menschen, mit einer Temperiervorrichtung (1) nach einem der Ansprüche 1 bis 13, wobei die Temperiervorrichtung (1) innerhalb der Vorrichtung angeordnet ist.

15. Anordnung (200) umfassend eine Auflagevorrichtung (201) für ein Lebewesen, insbesondere eine Krankentrage, und eine Temperiervorrichtung (1) nach einem der Ansprüche 1 bis 13, wobei das Wärmeelement (1) auf einer dem Lebewesen zugewandten Seite der Auflagevorrichtung (201) angeordnet ist.
